# EUROPEAN PATENT SPECIFICATION

(11) **EP 4 319 626 B1**
(45) Date of publication and mention of the grant of the patent: **14.10.2026**
(21) Application number: 22785127.6
(22) Date of filing: 23.02.2022
(51) Int. Cl.: A61B 5/02, A61B 5/021, A61B 5/026, A61B 5/022, A61B 5/024, A61B 5/00, G16H 40/63, G16H 50/20, G16H 50/30

(54) **SYSTEM AND METHOD FOR CORRELATING PULSE OXIMETRY WAVEFORM SIGNALS WITH BLOOD PRESSURE**
SYSTEM UND VERFAHREN ZUR KORRELATION VON PULSOXIMETRIEWELLENFORMSIGNALEN MIT DEM BLUTDRUCK
SYSTÈME ET PROCÉDÉ DE CORRÉLATION DE SIGNAUX DE FORME D'ONDE D'OXYMÉTRIE DE POULS AVEC UNE PRESSION ARTÉRIELLE

(30) Priority: 08.04.2021 US 202163172270 P; 07.10.2021 US 202117496052
(43) Date of publication of application: 14.02.2024
(73) Proprietor: Curtis, Guy, San Diego, CA 92110 (US)
(72) Inventor: Curtis, Guy, San Diego, CA 92110 (US)
(74) Representative: Tomkins & Co
(86) International application number: PCT/US2022/017443
(87) International publication number: WO 2022/216376

(56) References cited:
- US-A- 5 913 826
- US-A1- 2006 025 694
- US-A1- 2010 249 616
- US-A1- 2012 277 602
- US-A1- 2013 296 671
- US-A1- 2015 051 463
- US-A1- 2018 055 389
- CANNESSON MAXIME; BESNARD CYRIL; DURAND PIERRE G; BOHé JULIEN; JACQUES DIDIER: "Relation between respiratory variations in pulse oximetry plethysmographic waveform amplitude and arterial pulse pressure in ventilated patients", CRITICAL CARE, BIOMED CENTRAL LTD LONDON, GB, vol. 9, no. 5, 23 August 2005 (2005-08-23), GB , pages R562 - R568, XP021012397, ISSN: 1364-8535, DOI: 10.1186/cc3799
- FABIAN SCHRUMPF; PATRICK FRENZEL; CHRISTOPH AUST; GEORG OSTERHOFF; MIRCO FUCHS: "Assessment of deep learning based blood pressure prediction from PPG and rPPG signals", ARXIV.ORG, CORNELL UNIVERSITY LIBRARY, 201 OLIN LIBRARY CORNELL UNIVERSITY ITHACA, NY 14853, 15 April 2021 (2021-04-15), 201 Olin Library Cornell University Ithaca, NY 14853 , XP081940768

## Description

### RELATED APPLICATIONS

This application claims the benefit of U.S. Provisional Patent Application Serial No. 63/172,270, filed April 8, 2021.

### FIELD OF THE INVENTION

The present invention pertains generally to blood pressure and blood flow monitoring systems. More particularly, the present invention pertains to systems and methods that continuously provide comprehensive information regarding the efficacy of a patient's heart muscle function. The present invention is particularly, but not exclusively, useful for periodically calibrating an oximeter with contemporaneous blood pressure measurements, taken by a sphygmomanometer, to continuously display information from the oximeter regarding a patient's heart rate, blood flow and blood pressure in a clinical environment.

### BACKGROUND OF THE INVENTION

An oximeter is a medical device that is well known for its ability to accurately indicate a patient's local blood flow characteristics. Specifically, an oximeter can record the sinusoidal characteristics of a blood flow waveform that provide both temporal and amplitude values. Of particular concern for the present invention are the magnitudes of sequential peak amplitudes and the time interval between these peak amplitudes in the blood flow waveform. With this information, a patient's heart rate and blood flow volume can be determined. These characteristics alone, however, do not indicate another important physical measurement, namely: blood pressure.

In a clinical environment it is important to have as much timely information as possible, for both a patient's blood flow and for his/her blood pressure. Collectively, this information is both interdependent and interrelated. However, unlike an oximeter which can be automatically operated continuously to record a blood flow waveform, the operation of a sphygmomanometer to measure blood pressure is labor intensive and can be realistically employed only intermittently. Heretofore, this operational disconnect has, for the most part, been tolerated.

A consequence of the interrelationship between the blood flow waveform and blood pressure is the fact there are three separately measurable characteristics of particular importance. These characteristics are variable and include: 1) the peak amplitude A of a pulse in the blood flow waveform; 2) the time interval Δt between these peak amplitudes (i.e., "heart rate"); and 3) blood pressure P. When considered together, collectively, these variables can lead to clinical conclusions that might otherwise have been missed. An exemplary system for continuously monitoring blood pressure and blood flow in the vasculature of a subject are known from document US 2015/051463 A1.

With the above in mind, it is an object of the present invention to provide an oximeter which can be periodically calibrated, using a sphygmomanometer, to simultaneously provide continuous blood pressure readings with blood flow waveform information. Yet another object of the present invention is to incorporate heart rate information (±Δt) together with blood flow data (±ΔA) and blood pressure measurements (±ΔP) to provide a more comprehensive display presentation for clinical personnel with which to assess a patient's condition. Still another object of the present invention is to conduct continuous noninvasive blood pressure monitoring to elucidate new data concerning normal and abnormal states to greatly increase the diagnostic power and patient safety in the clinical environment. Another object of the present invention is to provide a system for continuously monitoring a patient's clinical condition which is easy to install, is simple to operate and which is comparatively cost effective.

### SUMMARY OF THE INVENTION

The invention is defined by the appended claims. In accordance with the present invention, a system and method for continuously monitoring blood pressure in the vasculature of a patient requires a comparative interface between blood flow and blood pressure in the patient's vasculature. To do this, it is necessary to initially establish a steady-state condition for the relationship between a patient's blood flow measurement A and his/her blood pressure reading P. This steady-state condition is then used as input for a computer. In an operation of the system, the patient's blood flow waveform is continuously monitored, and continuously compared with a predetermined operational model. Based on this comparison, the blood flow waveform is used as a basis for displaying a corresponding blood pressure reading for the patient.

To establish a steady-state condition, a sphygmomanometer is used to obtain a blood pressure reading P_{measured}. As is common practice, P_{measured} defines the difference between a P_{systolic} pressure and a P_{diastolic} pressure. This blood pressure reading P_{measured} is then used to calibrate a blood flow measurement A_{calibrated}. More specifically, A_{calibrated} is concurrently obtained by an oximeter together with P_{measured} from a sphygmomanometer. Importantly, P_{measured} and A_{calibrated} are established simultaneously while the patient is in a steady state condition. The respectively identified P_{measured} and A_{calibrated} are then used as computer input to establish the patient's steady state condition for a computer operation.

After it has been calibrated, the oximeter is used to continuously monitor a local blood flow waveform of the patient. This waveform is typically sinusoidal, with each pulse in the waveform having a unique peak amplitude A. Also, the waveform will have a time interval Δt between the peak amplitude of each pulse and the peak amplitude of the immediately preceding pulse in the waveform. Thus, the waveform identifies a computer input that includes both a heart rate based on Δt and a blood flow volume based on both Δt and A. With this information the computer then employs a predetermined operational model that correlates changes in blood flow ±ΔA with changes in blood pressure ±ΔP.

In detail, for a constant blood flow condition, the predetermined operational model can be mathematically expressed as A = P/R, where R is a factor representing the patient's vascular resistance to blood flow. In this ratio relationship, changes in blood flow ±ΔA and changes in blood pressure ±ΔP are equated to each other for correlation purposes as ±ΔA/A_{calibrated} ≈ ±ΔP/P_{measured}. Importantly, the correlation of blood flow with blood pressure in this operational model is made relative to the previously established steady-state condition. For this purpose the steady state condition can be expressed as (ΔP)base = P_{systolic} - P_{diastolic}.

For the present invention, a display unit is provided for displaying blood pressure variations ±ΔP corresponding to variations in the blood flow waveform ±ΔA. As noted above, this correspondence is made in accordance with the operational model having a constant (ΔP)_{base}. Also, for the purpose of assessing blood flow in the patient's vasculature, the display unit will show whether there are any consequent changes in heart rate ±Δt that are associated with concurrently measured ±ΔA. Specifically, the display unit selectively presents ±ΔP in the context of either a first operational state wherein Δt is constant, or a second operational state wherein Δt is variable.

In the first operational state, Δt is constant and, to maintain a proper mathematically constant blood flow relationship, R is varied whenever A is varied in the operational model A = P/R. Specifically, with a +ΔA there will be a comparable change in R >1, and with a -ΔA there will be a comparable change in R < 1. In the short term, this relationship can be considered valid because R is anatomically slow to vary. On the other hand, R may become a factor over a relatively longer term.

In the second operational state, Δt is variable, and R remains constant to maintain the operational relationship between ±ΔA and ±ΔP. Despite this fact, ±ΔA and ±ΔP may vary somewhat. In this latter case, recalibration may be necessary. Thus, in each operational state the operational model will determine a blood pressure P for display that may include important clinical information pertinent to the patient's condition.

Additional features for the present invention envision the use of a monitor for recording variations of ±ΔA and ±Δt in the blood flow waveform during a predetermined time duration. These measurements can then be compared with earlier measurements to determine whether ±ΔA and ±Δt have sufficiently stabilized during the predetermined time duration to identify a new value for the blood flow (e.g., A'). If so, the steady state condition should be recalibrated. For this purpose, the present invention envisions using a sphygmomanometer to periodically obtain new blood pressure readings P'_{measured} to recalibrate a new value for the blood flow A' as A'_{calibrated} for use with P'_{measured} to identify a new steady state condition for the patient.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a perspective view of the system for the present invention shown connected to a patient in a clinical environment;
Fig. 2 is graphical presentation of a patient's blood flow waveform;
Fig. 3 is a graphical presentation of a tracing profile for the patient's blood flow; and
Fig. 4 is a schematic presentation of the operational components for the present invention showing their interactive connections with each other for an operation of the present invention.

### DESCRIPTION OF THE PREFERRED EMBODIMENTS

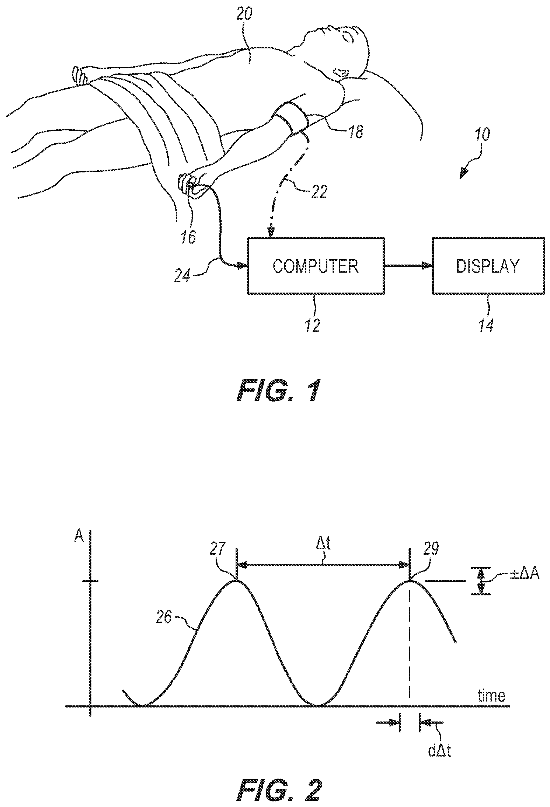

Referring initially to Fig. 1, a system for taking a blood pressure reading in accordance with the present invention is shown and is generally designated 10. As shown, the system 10 includes a computer 12 which is connected to a display 14. An oximeter 16 and a sphygmomanometer cuff 18 are typically placed on a patient 20 as shown in Fig. 1. The dot-dash line 22 connecting the sphygmomanometer cuff 18 with the computer 12 is shown differentiated to thereby indicate that this connection is not continuous. Instead, as is disclosed below, the sphygmomanometer cuff 18 is used primarily for the purpose of periodically calibrating the oximeter 16. On the other hand, the solid line 24 is shown to indicate a permanent connection between the oximeter 16 and the patient 20.

The purposes of the oximeter 16 and the sphygmomanometer cuff 18 are respectively of a type well known in the industry. Specifically, the sphygmomanometer cuff 18 is used to take periodic blood pressure readings, P, from the patient 20 that will include both a systolic pressure, P_{systolic}, and a diastolic pressure, P_{diastolic.} The oximeter 16, on the other hand, is used to identify a blood flow waveform 26 such as is shown in Fig. 2.

In Fig. 2 it is to be appreciated that all blood flow waveforms 26 are essentially sinusoidal and they have certain characteristics in common. Of these, the characteristics of particular interest for the present invention are changes in the magnitude ΔA of successive peaks in the waveform 26 and the time duration Δt between successive peaks. Fig. 2 shows these variable characteristics with reference to the peaks 27 and 29. Importantly, both variables (ΔA and Δt) are measured by the oximeter 16.

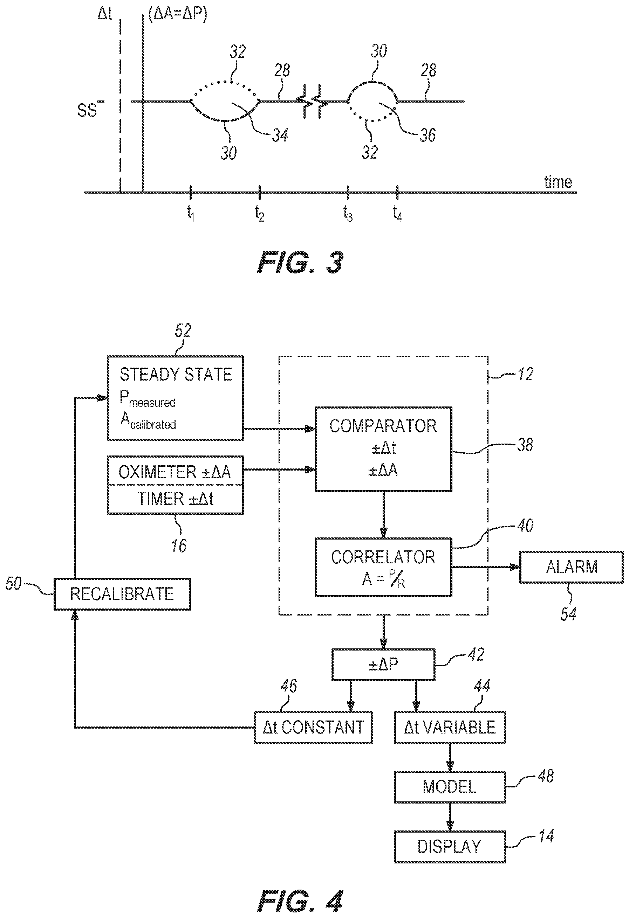

A consequence of changes in the respective magnitudes of ΔA and Δt is that as one increases the other typically decreases. As disclosed above, for purposes of the present invention, a steady state is established when the patient 20 is resting. With the patient 20 resting, a base relationship is created where A = P/R is constant, and R = 1. In this base relationship (ΔA/sec)_{base} = (ΔP)_{base} = 0. Nevertheless, when the patient 20 has a short-term episode with a ±ΔA the relationship ΔA = ΔP is still considered acceptable, at least in the short term. The consequence of this is best appreciated with reference to Fig. 3.

Examples for an operation of the present invention are provided below. In both of these examples, the steady state case is presented when R, in the expression A = P/R, is constant, with R = 1. This situation is such that (ΔA/Δt)_{base} = ΔP/R » ±ΔP. Also, it must be appreciated that (ΔP)_{base} will be determined on a case-by-case basis and will vary accordingly.

By way of example, consider an operational model being established where (ΔP)_{base} = ΔP_{systolic} - ΔP_{diastolic} = 120 - 60 = 60 and where, ΔP_{systolic} is approximated at 4/5ΔP, and ΔP_{diastolic} is approximated at 1/5ΔP. Moreover, it is important to recognize that when there is an increase in Δt there will be a drop in P. And vice-versa, when there is a decrease in Δt there will be a rise in P.

### Example 1 (Increase in Heart Rate Δt)

In accordance with the expression A= P/R, for an increase in heart rate by a factor of 2 (i.e., Δt decreases by ½), R in the expression A = P/R must also be considered equal to 2. Thus, ΔP = (ΔP)_{base}/2 = 30 for a decrease in ΔP. Thus, for this example, (ΔP)_{display} will equal [120 - 4/5(30)] / [60 - 1/5(30)] = 96/54.

### Example 2 (Decrease in Heart Rate Δt)

In accordance with the expression A= P/R, for a decrease in heart rate by a factor of 15% (i.e., Δt increases by a factor of 1/.85 = 1.18), R in the expression A=P/R must also be considered equal to 0.85. Thus, ΔP = (ΔP)_{base}/0.85 = 60/.85 = 70.1) for an increase in ΔP. Thus, for this example, (ΔP)_{display} will equal [120 + 4/5(70.1)] / [60 + 1/5(70.1)] = 176/74.

In Fig. 3, a tracing profile 28 is shown for the waveform 26. As indicated in Fig. 3, the tracing profile 28 has two different characteristic components, i.e., Δt and ΔA. These characteristic components are individually shown respectively in Fig. 3 as a dashed line 30 for Δt, and a dotted line 32 for ΔA. In a steady state (SS) condition for the waveform 26, i.e., when ΔA/Δt is considered to be constant, the tracing profile 28 is shown by a solid line. Fig. 3 also shows that in keeping with the expression A = P/R, ΔA ≈ ΔP when these variables are in the steady state, SS.

Still referring to Fig. 3, examples for two different episodes of clinical interest for the present invention are shown. One is an increased pressure episode 34 for the patient 20, and the other is a decreased pressure episode 36 for the patient 20. During an increased pressure episode 34, Δt will decrease (i.e., heart rate increases) as indicated by dashed line 30. Concomitantly, the display 14 will show an increase in ΔP. On the other hand, during a decreased pressure episode 36, Δt will increase (i.e., heart rate will decrease) and ΔP will decrease. As recognized by the present invention, Δt and ΔA (a.k.a. ΔP) will both be measured by the oximeter 16.

With reference to Fig. 4, it will be seen that the computer 12 includes a comparator 38 and a correlator 40. As shown, the comparator 38 receives information from the oximeter 16 regarding both ±ΔA and ±Δt. These variables are then correlated by the correlator 40 to determine an output ±ΔP that is analyzed as indicated by the action block 42. Specifically, ±ΔP can be selectively analyzed relative to either a variable Δt at action block 44, or relative to a constant Δt (i.e., Δt = 0) at action block 46.

In the circumstance when Δt is variable (block 44) the model 48 for P_{base} is evaluated as disclosed above. As also disclosed above, the result of this evaluation is shown on the display 14. On the other hand, when Δt is considered a constant (block 46) the variable R is evaluated for a possible recalibration (block 50) of the expression A = P/R or for sounding an alarm 54. In the event recalibration is implemented, the steady state SS for P_{measured} and A_{calibrated} with a new (ΔP)_{base} is established using the oximeter 16 and the sphygmomanometer 18. This action is indicated by action block 52.

While the particular System and Method for Correlating Pulse Oximetry Waveform Signals with Blood Pressure as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system (10) for continuously monitoring blood pressure in the vasculature of a patient which comprises:
an oximeter (16) configured to be positioned on the patient and to measure a sinusoidal waveform representing a patient's local blood flow having a peak amplitude for each pulse in the waveform, and a time interval Δt between the peak amplitudes of sequential pulses in the waveform, to collectively identify a blood flow A,
a sphygmomanometer (18) for obtaining a blood pressure reading P for the patient, wherein P is defined as a difference between a P_{systolic} pressure and a P_{diastolic} pressure, the sphygmomanometer being configured to calibrate the oximeter when A and P are obtained simultaneously, in order to respectively identify an A_{calibrated} and a P_{measured} for use as computer input for the patient's steady state condition;
a computer (12) for receiving the blood flow measurement A and the A_{calibrated} and P_{measured} from the oximeter, wherein the computer is configured to employ an operational relationship expressed as A = P/R, wherein R is a factor representing a vascular resistance to the patient's blood flow A, to correlate changes in blood flow ±ΔA with changes in blood pressure ±ΔP relative to the steady state condition of the patient; and
a display unit (14) for displaying ±ΔP based on ±ΔA, and displaying whether there is any consequent ±Δt associated with the measured ±ΔA for assessing blood flow in the patient's vasculature.

2. The system of claim 1 wherein the computer employs a ratio relationship between changes in blood flow ±ΔA and changes in blood pressure ±ΔP, expressed as ±ΔA/A_{calibrated} = ±ΔP/P_{measured} for correlation purposes.

3. The system of claim 2 wherein the display unit is configured to selectively present ±ΔP in the context of:
a first operational state when Δt is constant, and R is variable to maintain the operational relationship A = P/R, with R > 1 for a +ΔA and R < 1 for a -ΔA; and
a second operational state when Δt is variable, and R is constant to maintain the operational relationship between ±ΔA and ±ΔP with R = 1.

4. The system of claim 3 wherein ΔP = ΔP_{systolic} - ΔP_{diastolic}, wherein ΔP_{systolic} is approximated as being 4/5ΔP, and wherein ΔP_{diastolic} is approximated as being 1/5ΔP.

5. The system of claim 4 wherein the computer comprises:
a timer for measuring Δt between sequential pulses in the blood flow waveform;
a comparator for comparing a preceding Δt with the peak amplitude of the immediately following pulse in the blood flow A; and
a correlator for analyzing Δt, together with ±ΔA for each pulse, to identify, for display, the operational state of blood flow A in the patient.

6. The system of claim 4, configured to periodically recalibrate a steady state condition for the patient in accordance with clinical requirements.

7. The system of claim 6 wherein the comparator further comprises a monitor for recording variations ±ΔA and ±Δt of the blood flow waveform during a predetermined period of time.

8. A system (10) for continuously monitoring blood flow characteristics in the vasculature of a patient which comprises:
an oximeter (16) configured to monitor a local blood flow waveform of the patient wherein the waveform is sinusoidal and each pulse in the waveform has a peak amplitude A with a time interval Δt between the peak amplitude of the pulse and the peak amplitude of the immediately preceding pulse in the waveform, to collectively identify a heart rate based on Δt and a blood flow volume based on both Δt and A;
a sphygmomanometer (18) configured to obtain a blood pressure reading P_{measured} and to calibrate A with the measured blood pressure reading P_{measured} for a sequence of pulses in the waveform, to identify an A_{calibrated}, wherein P_{measured} and A_{calibrated} are established simultaneously while the patient is in a steady state condition;
a computer (12) for continuously receiving data from the oximeter pertinent to variations in heart rate ±Δt and variations in peak amplitudes ±ΔA, to identify ±ΔA corresponding to changes in blood pressure ±ΔP based on a predetermined operational relationship which correlates changes in peak amplitudes ±ΔA as changes in blood pressure ±ΔP relative to the steady state of the patient, and further configured to evaluate variations in heart rate ±Δt in context with ±ΔA; and
a display unit (14) for displaying ±ΔP based on ±ΔA, and displaying whether there is any consequent ±Δt associated with the measured ±ΔA for assessing blood flow in the patient's vasculature.

9. The system of claim 8 wherein the predetermined operational relationship is expressed as A = P/R where R is a factor representing a vascular resistance to the patient's blood flow, and wherein ±ΔP is considered in the context of:
a first operational state when Δt is constant, and R is variable to maintain the operational relationship A = P/R, wherein with R > 1 there is a +ΔA, and with R < 1 there is a -ΔA; and
a second operational state when Δt is variable, and R is constant to maintain the operational relationship between ±ΔA and ±ΔP with R = 1.

10. The system of claim 9 further comprising a display unit configured to present P as a P_{systolic} = P_{measured} ±ΔP_{systolic}, and a P_{diastolic} = P_{measured} ± ΔP_{diastolic}, and further wherein ΔP = ΔP_{systolic} - ΔP_{diastolic}, wherein ΔP_{systolic} is approximated as being 4/5ΔP, and wherein ΔP_{diastolic} is approximated as being 1/5ΔP.

11. The system of claim 10, further comprising a monitor for recording variations of ±ΔA and ±Δt in the blood flow waveform during a predetermined time duration and wherein the sphygmomanometer is configured to obtain a new blood pressure reading P'_{measured} corresponding to A' to recalibrate a new value for the blood flow A as A'_{calibrated} for use with P'_{measured} to identify the patient's steady state condition.

12. A method for calibrating an oximeter to provide continuous blood pressure and heart function information from a patient in a clinical environment, which comprises the steps of:
creating an environment wherein the patient's heart muscle is stabilized to represent a steady state condition with a blood flow A;
obtaining a blood pressure reading P measured for the patient during the steady state condition, wherein P is defined as a difference between a systolic pressure P_{systolic} and a diastolic pressure P_{diastolic}, and P is designated P_{measured};
calibrating the patient's blood flow A with P_{measured} to establish a calibrated value A_{calibrated} for the patient's blood flow;
providing P_{measured} and A_{calibrated} as input for a computer;
monitoring an output from the oximeter at the computer to equate changes in blood flow ±ΔA relative to A_{calibrated} with changes in blood pressure ±ΔP relative to P_{measured} based on a ratio relationship where ±ΔA/A_{calibrated} ≈ ±ΔP/P_{measured}, and wherein the computer further employs an operational relationship expressed as A = P/R where R is a factor representing a vascular resistance to the patient's blood flow A, to correlate changes in blood flow ±ΔA with changes in blood pressure ±ΔP relative to the steady state condition of the patient; and
displaying P in the clinical environment as a P_{systolic} = P_{measured} ± ΔP_{systolic}, and a P_{diastolic} = P_{measured} ±ΔP_{diastolic}.

13. The method of claim 12 wherein A has a sinusoidal waveform representing a patient's local blood flow with a peak amplitude for each pulse in the waveform, and a time interval Δt between the peak amplitudes of sequential pulses in the waveform, to collectively identify the blood flow A.

14. The method of claim 13 wherein the displaying step further comprising the steps of:
evaluating whether there is any consequent ±Δt associated with the measured ±ΔA for assessing blood flow in the patient's vasculature;
presenting a first operational state when Δt is constant, and R is variable to maintain the operational relationship A = P/R, wherein with R > 1 there is a +ΔA, and with R < 1 there is a -ΔA; and
presenting a second operational state when Δt is variable, and R is constant to maintain the operational relationship between ±ΔA and ±ΔP with R = 1.

15. The method of claim 14 further comprising the steps of:
recording variations ±ΔA and ±Δt of the blood flow waveform during a predetermined period of time.

## Patentansprüche

1. Ein System (10) zum kontinuierlichen Überwachen des Blutdrucks in dem Gefäßsystem eines Patienten, das Folgendes aufweist:
ein Oximeter (16), das zum Positionieren an dem Patienten und zum Messen einer sinusförmigen Wellenform eingerichtet ist, die einen lokalen Blutfluss des Patienten darstellt und für jeden Puls in der Wellenform eine Spitzenamplitude sowie ein Zeitintervall Δt zwischen den Spitzenamplituden aufeinanderfolgender Pulse in der Wellenform aufweist, um gemeinsam einen Blutfluss A zu identifizieren;
ein Blutdruckmessgerät (18) zum Erhalten eines Blutdruckmesswerts P für den Patienten, wobei P als eine Differenz zwischen einem Druck P_{systolisch} und einem Druck P_{diastolisch} definiert ist, wobei das Blutdruckmessgerät dazu eingerichtet ist, das Oximeter zu kalibrieren, wenn A und P gleichzeitig erhalten werden, um jeweils ein A_{kalibriert} und ein P_{gemessen} zur Verwendung als Computereingabe für einen Steady-State-Zustand des Patienten zu identifizieren;
einen Computer (12) zum Empfangen der Blutflussmessung A sowie des A_{kalibriert} und des P_{gemessen} von dem Oximeter, wobei der Computer dazu eingerichtet ist, eine Betriebsbeziehung anzuwenden, die als A = P/R ausgedrückt ist, wobei R ein einen Gefäßwiderstand gegenüber dem Blutfluss A des Patienten darstellender Faktor ist, um Änderungen des Blutflusses ±ΔA mit Änderungen des Blutdrucks ±ΔP relativ zum Steady-State-Zustand des Patienten zu korrelieren; und
eine Anzeigeeinheit (14) zum Anzeigen von ±ΔP basierend auf ±ΔA und zum Anzeigen, ob ein daraus resultierendes ±Δt mit dem gemessenen ±ΔA verbunden ist, um den Blutfluss in dem Gefäßsystem des Patienten zu beurteilen.

2. Das System nach Anspruch 1, wobei der Computer eine Verhältnisbeziehung zwischen Änderungen des Blutflusses ±ΔA und Änderungen des Blutdrucks ±ΔP anwendet, die für Korrelationszwecke als ±ΔA/A_{kalibriert} ≈ ±ΔP/P_{gemessen} ausgedrückt ist.

3. Das System nach Anspruch 2, wobei die Anzeigeeinheit dazu eingerichtet ist, ±ΔP selektiv im Kontext von Folgendem darzustellen:
einem ersten Betriebszustand, wenn Δt konstant ist und R variabel ist, um die Betriebsbeziehung A = P/R aufrechtzuerhalten, wobei R > 1 für ein +ΔA und R < 1 für ein -ΔA gilt; und
einem zweiten Betriebszustand, wenn Δt variabel ist und R konstant ist, um die Betriebsbeziehung zwischen ±ΔA und ±ΔP mit R = 1 aufrechtzuerhalten.

4. Das System nach Anspruch 3, wobei ΔP = ΔP_{systolisch} - ΔP_{diastolisch}, wobei ΔP_{systolisch} näherungsweise als 4/5ΔP angenommen wird und wobei ΔP_{diastolisch} näherungsweise als 1/5ΔP angenommen wird.

5. Das System nach Anspruch 4, wobei der Computer Folgendes aufweist:
einen Zeitgeber zum Messen von Δt zwischen aufeinanderfolgenden Pulsen in der Blutfluss-Wellenform;
einen Komparator zum Vergleichen eines vorhergehenden Δt mit der Spitzenamplitude des unmittelbar folgenden Pulses in dem Blutfluss A; und
einen Korrelator zum Analysieren von Δt zusammen mit ±ΔA für jeden Puls, um, und zwar zur Anzeige, den Betriebszustand des Blutflusses A in dem Patienten zu identifizieren.

6. Das System nach Anspruch 4, das dazu eingerichtet ist, einen Steady-State-Zustand für den Patienten periodisch gemäß klinischen Anforderungen neu zu kalibrieren.

7. Das System nach Anspruch 6, wobei der Komparator ferner ein Überwachungsgerät zum Aufzeichnen von Änderungen ±ΔA und ±Δt der Blutfluss-Wellenform während eines vorbestimmten Zeitraums aufweist.

8. Ein System (10) zum kontinuierlichen Überwachen von Blutflusseigenschaften in dem Gefäßsystem eines Patienten, das Folgendes aufweist:
ein Oximeter (16), das dazu eingerichtet ist, eine lokale Blutfluss-Wellenform des Patienten zu überwachen, wobei die Wellenform sinusförmig ist und jeder Puls in der Wellenform eine Spitzenamplitude A mit einem Zeitintervall Δt zwischen der Spitzenamplitude des Pulses und der Spitzenamplitude des unmittelbar vorhergehenden Pulses in der Wellenform aufweist, um gemeinsam eine Herzfrequenz basierend auf Δt und ein Blutflussvolumen basierend sowohl auf Δt als auch auf A zu identifizieren;
ein Blutdruckmessgerät (18), das dazu eingerichtet ist, einen Blutdruckmesswert P_{gemessen} zu erhalten und A mit dem gemessenen Blutdruckmesswert P_{gemessen} für eine Folge von Pulsen in der Wellenform zu kalibrieren, ein A_{kalibriert} zu identifizieren, wobei P_{gemessen} und A_{kalibriert} gleichzeitig festgelegt werden, während sich der Patient in einem Steady-State-Zustand befindet;
einen Computer (12) zum kontinuierlichen Empfangen von Daten von dem Oximeter, die Änderungen der Herzfrequenz ±Δt und Änderungen der Spitzenamplituden ±ΔA betreffen, um ein ±ΔA zu identifizieren, das Änderungen des Blutdrucks ±ΔP entspricht, basierend auf einer vorbestimmten Betriebsbeziehung, die Änderungen der Spitzenamplituden ±ΔA als Änderungen des Blutdrucks ±ΔP relativ zum Steady-State-Zustand des Patienten korreliert, und der ferner dazu eingerichtet ist, Änderungen der Herzfrequenz ±Δt im Kontext mit ±ΔA auszuwerten; und
eine Anzeigeeinheit (14) zum Anzeigen von ±ΔP basierend auf ±ΔA und zum Anzeigen, ob ein daraus resultierendes ±Δt mit dem gemessenen ±ΔA verbunden ist, um den Blutfluss in dem Gefäßsystem des Patienten zu beurteilen.

9. Das System nach Anspruch 8, wobei die vorbestimmte Betriebsbeziehung als A = P/R ausgedrückt ist, wobei R ein einen Gefäßwiderstand gegenüber dem Blutfluss des Patienten darstellender Faktor ist, und wobei ±ΔP im Kontext von Folgendem betrachtet wird:
einem ersten Betriebszustand, wenn Δt konstant ist und R variabel ist, um die Betriebsbeziehung A = P/R aufrechtzuerhalten, wobei bei R > 1 ein +ΔA vorliegt und bei R < 1 ein -ΔA vorliegt; und
einem zweiten Betriebszustand, wenn Δt variabel ist und R konstant ist, um die Betriebsbeziehung zwischen ±ΔA und ±ΔP mit R = 1 aufrechtzuerhalten.

10. Das System nach Anspruch 9, das ferner eine Anzeigeeinheit aufweist, die dazu eingerichtet ist, P als ein P_{systolisch} = P_{gemessen} ±ΔP_{systolisch}, und ein P_{diastolisch} = P_{gemessen} ±ΔP_{diastolisch}, und wobei ferner ΔP = ΔP_{systolisch} - ΔP_{diastolisch}, wobei ΔP_{systolisch} näherungsweise als 4/5ΔP angenommen wird und wobei ΔP_{diastolisch} näherungsweise als 1/5ΔP angenommen wird.

11. Das System nach Anspruch 10, das ferner ein Überwachungsgerät zum Aufzeichnen von Änderungen ±ΔA und ±Δt in der Blutfluss-Wellenform während einer vorbestimmten Zeitdauer aufweist, und wobei das Blutdruckmessgerät dazu eingerichtet ist, einen neuen Blutdruckmesswert P'_{gemessen} entsprechend A' zu erhalten, um einen neuen Wert für den Blutfluss A als A'_{kalibriert} zur Verwendung mit P'_{gemessen} neu zu kalibrieren, um den Steady-State-Zustand des Patienten zu identifizieren.

12. Ein Verfahren zum Kalibrieren eines Oximeters zum Bereitstellen kontinuierlicher Blutdruck- und Herzfunktionsinformationen von einem Patienten in einer klinischen Umgebung, das die folgenden Schritte aufweist:
Schaffen einer Umgebung, in der der Herzmuskel des Patienten stabilisiert wird, um einen Steady-State-Zustand mit einem Blutfluss A darzustellen;
Erhalten eines Blutdruckmesswerts P gemessen für den Patienten während des Steady-State-Zustands, wobei P als eine Differenz zwischen einem systolischen Druck P_{systolisch} und einem diastolischen Druck P_{diastolisch} definiert ist und P als P_{gemessen} bezeichnet wird;
Kalibrieren des Blutflusses A des Patienten mit P_{gemessen}, um einen kalibrierten Wert A_{kalibriert} für den Blutfluss des Patienten festzulegen;
Bereitstellen von P_{gemessen} und A_{kalibriert} als Eingabe für einen Computer;
Überwachen einer Ausgabe des Oximeters an dem Computer, um Änderungen des Blutflusses ±ΔA relativ zu A_{kalibriert} mit Änderungen des Blutdrucks ±ΔP relativ zu P_{gemessen} basierend auf einer Verhältnisbeziehung gleichzusetzen, wobei ±ΔA/A_{kalibriert} ≈ ±ΔP/P_{gemessen}, und wobei der Computer ferner eine Betriebsbeziehung anwendet, die als A = P/R ausgedrückt ist, wobei R ein einen Gefäßwiderstand gegenüber dem Blutfluss A des Patienten darstellender Faktor ist, um Änderungen des Blutflusses ±ΔA mit Änderungen des Blutdrucks ±ΔP relativ zum Steady-State-Zustand des Patienten zu korrelieren; und
Anzeigen von P in der klinischen Umgebung als ein ${P}_{systolisch} = {P}_{gemessen} \pm {ΔP}_{systolisch} , und ein {P}_{diastolisch} = {P}_{gemessen} \pm {ΔP}_{diastolisch} .$

13. Das Verfahren nach Anspruch 12, wobei A eine sinusförmige Wellenform hat, die einen lokalen Blutfluss des Patienten mit einer Spitzenamplitude für jeden Puls in der Wellenform und einem Zeitintervall Δt zwischen den Spitzenamplituden aufeinanderfolgender Pulse in der Wellenform darstellt, um gemeinsam den Blutfluss A zu identifizieren.

14. Das Verfahren nach Anspruch 13, wobei der Schritt des Anzeigens ferner die folgenden Schritte aufweist:
Auswerten, ob ein daraus resultierendes ±Δt mit dem gemessenen ±ΔA verbunden ist, um den Blutfluss in dem Gefäßsystem des Patienten zu beurteilen;
Darstellen eines ersten Betriebszustands, wenn Δt konstant ist und R variabel ist, um die Betriebsbeziehung A = P/R aufrechtzuerhalten, wobei bei R > 1 ein +ΔA vorliegt und bei R < 1 ein -ΔA vorliegt; und
Darstellen eines zweiten Betriebszustands, wenn Δt variabel ist und R konstant ist, um die Betriebsbeziehung zwischen ±ΔA und ±ΔP mit R = 1 aufrechtzuerhalten.

15. Das Verfahren nach Anspruch 14, das ferner die folgenden Schritte aufweist:
Aufzeichnen von Änderungen ±ΔA und ±Δt der Blutfluss-Wellenform während eines vorbestimmten Zeitraums.

## Revendications

1. Un système (10)
pour la surveillance continue de la pression artérielle dans le système vasculaire d'un patient, dans lequel comprend :
un oxymètre (16)
configuré pour être positionné sur le patient et pour mesurer une forme d'onde sinusoïdale représentant un flux sanguin local du patient ayant une amplitude de crête pour chaque impulsion dans la forme d'onde, et un intervalle de temps Δt entre les amplitudes de crête d'impulsions séquentielles dans la forme d'onde, afin d'identifier collectivement un flux sanguin A,
un sphygmomanomètre (18)
pour obtenir une mesure de la pression artérielle P pour le patient, dans laquelle P est définie comme une différence entre une pression P_{systolique} et une pression P_{diastolique}, le sphygmomanomètre étant configuré pour étalonner l'oxymètre lorsque A et P sont obtenus simultanément, afin d'identifier respectivement un A_{calibrated} et un P_{measured} à utiliser comme entrée d'ordinateur pour l'état stationnaire du patient ;
un ordinateur (12)
pour recevoir la mesure de flux sanguin A ainsi que A_{calibrated} et P_{measured} provenant de l'oxymètre, dans lequel ordinateur est configuré pour employer une relation opérationnelle exprimée comme A = P/R, dans lequel R est un facteur représentant une résistance vasculaire au flux sanguin A du patient, pour corréler des variations du flux sanguin ±ΔA avec des variations de la pression artérielle ±ΔP par rapport à l'état stationnaire du patient ; et
une unité d'affichage (14)
pour l'affichage de ±ΔP basé sur ±ΔA, et l'affichage de l'existence d'un éventuel ±Δt associé à la ±ΔA mesurée pour l'évaluation du flux sanguin dans le système vasculaire du patient.

2. Le système selon la revendication 1, dans lequel l'ordinateur emploie une relation de rapport entre les variations du flux sanguin ±ΔA et les variations de la pression artérielle ±ΔP, exprimée comme ±ΔA / A_{calibrated} ≈ ±ΔP / P_{measured} à des fins de corrélation.

3. Le système selon la revendication 2, dans lequel l'unité d'affichage est configurée pour présenter sélectivement ±ΔP dans le contexte de :
un premier état opérationnel lorsque Δt est constant, et R est variable pour maintenir la relation opérationnelle A = P / R, avec R > 1 pour un +ΔA et R < 1 pour un -ΔA ; et
un second état opérationnel lorsque Δt est variable, et R est constant pour maintenir la relation opérationnelle entre ±ΔA et ±ΔP avec R = 1.

4. Le système selon la revendication 3, dans lequel ΔP = ΔPₛyₛₜₒlᵢc - Δ P_{diastolique}, dans lequel Δ P_{systolique} est approximé comme étant 4/5ΔP, et dans lequel Δ P_{diastolique} est approximé comme étant 1/5ΔP.

5. Le système selon la revendication 4, dans lequel l'ordinateur comprend :
un temporisateur pour mesurer Δt entre des impulsions séquentielles dans la forme d'onde du flux sanguin ;
un comparateur pour la comparaison d'un Δt précédent avec l'amplitude de crête de l'impulsion immédiatement suivante dans le flux sanguin A ; et
un corrélateur pour l'analyse de Δt, conjointement avec ±ΔA pour chaque impulsion, afin d'identifier, pour l'affichage, l'état opérationnel du flux sanguin A chez le patient.

6. Le système selon la revendication 4, configuré pour réétalonner périodiquement un état stationnaire pour le patient conformément aux exigences cliniques.

7. Le système selon la revendication 6, dans lequel le comparateur comprend en outre un moniteur pour l'enregistrement des variations ±ΔA et ±Δt de la forme d'onde du flux sanguin pendant une période de temps prédéterminée.

8. Un système (10)
pour la surveillance continue des caractéristiques du flux sanguin dans le système vasculaire d'un patient, dans lequel comprend :
un oxymètre (16)
configuré pour surveiller une forme d'onde de flux sanguin local du patient.
dans lequel la forme d'onde est sinusoïdale et chaque impulsion dans la forme d'onde présente une amplitude de crête A avec un intervalle de temps Δt entre l'amplitude de crête de l'impulsion et l'amplitude de crête de l'impulsion immédiatement précédente dans la forme d'onde, afin d'identifier collectivement une fréquence cardiaque basée sur Δt et un volume de flux sanguin basé à la fois sur Δt et A ;
un sphygmomanomètre (18)
configuré pour obtenir une mesure de la pression artérielle
P_{measured} et pour étalonner A avec la mesure de la pression artérielle P_{measured} pour une séquence d'impulsions dans la forme d'onde, afin d'identifier un A_{calibrated}, dans lequel P_{measured} et A_{calibrated} sont établis simultanément pendant que le patient est dans un état stationnaire ;
un ordinateur (12)
pour recevoir en continu des données provenant de l'oxymètre relatives à
des variations de la fréquence cardiaque ±Δt et des variations des amplitudes de crête ±ΔA, afin d'identifier ±ΔA correspondant à des variations de la pression artérielle ±ΔP sur la base d'une relation opérationnelle prédéterminée qui corrèle les variations des amplitudes de crête ±ΔA comme des variations de la pression artérielle ±ΔP par rapport à l'état stationnaire du patient, et en outre configuré pour évaluer les variations de la fréquence cardiaque ±Δt en contexte avec ±ΔA ; et
une unité d'affichage (14)
pour l'affichage de ±ΔP basé sur ±ΔA, et l'affichage de l'existence d'un éventuel ±Δt associé à la ±ΔA mesurée pour l'évaluation du flux sanguin dans le système vasculaire du patient.

9. Le système selon la revendication 8, dans lequel la relation opérationnelle prédéterminée est exprimée comme A = P / R, où R est un facteur représentant une résistance vasculaire au flux sanguin du patient, et dans lequel ±ΔP est considéré dans le contexte de :
un premier état opérationnel lorsque Δt est constant, et R est variable pour maintenir la relation opérationnelle A = P / R, dans lequel avec R > 1 il y a un +ΔA, et avec R < 1 il y a un -ΔA ; et
un second état opérationnel lorsque Δt est variable, et R est constant pour maintenir la relation opérationnelle entre ±ΔA et ±ΔP avec R = 1.

10. Le système selon la revendication 9, comprenant en outre une unité d'affichage configurée pour présenter P comme un P_{systolique} = P_{measured} ±Δ P_{systolique}, et un P_{diastolique} = P_{measured} ±Δ P_{diastolique}, et en outre dans lequel ΔP = Δ P_{systolique} - Δ P_{diastolique}, dans lequel Δ P_{systolique} est approximé comme étant 4/5ΔP, et dans lequel Δ P_{diastolique} est approximé comme étant 1/5ΔP.

11. Le système selon la revendication 10, comprenant en outre un moniteur pour l'enregistrement des variations de ±ΔA et ±Δt dans la forme d'onde du flux sanguin pendant une durée prédéterminée et dans lequel le sphygmomanomètre est configuré pour obtenir une nouvelle mesure de la pression artérielle P'_{measured} correspondant à A' pour réétalonner une nouvelle valeur du flux sanguin A comme A'_{calibrated} à utiliser avec P'_{measured} pour identifier l'état stationnaire du patient.

12. Un procédé pour étalonner un oxymètre afin de fournir des informations continues sur la pression artérielle et la fonction cardiaque d'un patient dans un environnement clinique, dans lequel comprend les étapes consistant à :
créer un environnement dans lequel le muscle cardiaque du patient est stabilisé pour représenter un état stationnaire avec un flux sanguin A ;
obtenir une mesure de la pression artérielle P mesurée pour le patient pendant l'état stationnaire, dans lequel P est définie comme une différence entre une pression systolique P_{systolique} et une pression diastolique P_{diastolique}, et P est désignée P_{measured};
étalonner le flux sanguin A du patient avec P_{measured} pour établir une valeur étalonnée A_{calibrated} pour le flux sanguin du patient ;
fournir P_{measured} et A_{calibrated} comme entrée pour un ordinateur ;
surveiller une sortie de l'oxymètre à l'ordinateur pour égaler les variations du flux sanguin ±ΔA par rapport à A_{calibrated} avec les variations de la pression artérielle ±ΔP par rapport à P_{measured} sur la base d'une relation de rapport où ±ΔA / A_{calibrated} ≈ ±ΔP / P_{measured}, et dans lequel l'ordinateur emploie en outre une relation opérationnelle exprimée comme A = P / R, où R est un facteur représentant une résistance vasculaire au flux sanguin A du patient, pour corréler des variations du flux sanguin ±ΔA avec des variations de la pression artérielle ±ΔP par rapport à l'état stationnaire du patient ; et
affichage de P dans l'environnement clinique comme un P_{systolique} = P_{measured} ±Δ P_{systolique}, et un P_{diastolique} = P_{measured} ±Δ P_{diastolique}.

13. Le procédé selon la revendication 12, dans lequel A présente une forme d'onde sinusoïdale représentant un flux sanguin local d'un patient avec une amplitude de crête pour chaque impulsion dans la forme d'onde, et un intervalle de temps Δt entre les amplitudes de crête d'impulsions séquentielles dans la forme d'onde, afin d'identifier collectivement le flux sanguin A.

14. Le procédé selon la revendication 13, dans lequel l'étape d'affichage comprend en outre les étapes consistant à :
évaluer s'il existe un éventuel ±Δt associé à la ±ΔA mesurée pour l'évaluation du flux sanguin dans le système vasculaire du patient ;
présenter un premier état opérationnel lorsque Δt est constant, et R est variable pour maintenir la relation opérationnelle A = P / R, dans lequel avec R > 1 il y a un +ΔA, et avec R < 1 il y a un -ΔA ; et
présenter un second état opérationnel lorsque Δt est variable, et R est constant pour maintenir la relation opérationnelle entre ±ΔA et ±ΔP avec R = 1.

15. Le procédé selon la revendication 14, comprenant en outre les étapes consistant à :
enregistrer les variations ±ΔA et ±Δt de la forme d'onde du flux sanguin pendant une période de temps prédéterminée.
